(19) Europäisches Patentamt
European Patent Office
Office européen des brevets

(11) **EP 2 434 278 A1**

(12) **EUROPEAN PATENT APPLICATION**

(43) Date of publication:
**28.03.2012 Bulletin 2012/13**

(51) Int Cl.:
*G01N 27/414* (2006.01)          *G01N 27/12* (2006.01)
*B82Y 15/00* (2011.01)

(21) Application number: **10012585.5**

(22) Date of filing: **30.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **31.08.2010 EP 10009053**

(71) Applicant: **Max-Planck-Gesellschaft zur Förderung der Wissenschaften e.V.
80539 München (DE)**

(72) Inventors:
• **Kurkina, Tetiana**
  **70565 Stuttgart (DE)**
• **Vlandas, Alexis**
  **70563 Stuttgart (DE)**
• **Rafiefard, Nassim**
  **70569 Stuttgart (DE)**
• **Ahmad, Ashraf**
  **70195 Stuttgart (DE)**
• **Kern, Klaus**
  **71111 Waldenbruch (DE)**
• **Balasubramanian, Kannan**
  **70565 Stuttgart (DE)**

(74) Representative: **Manitz, Finsterwald & Partner GbR
Postfach 31 02 20
80102 München (DE)**

(54) **Apparatus for detecting one or more analytes comprising an elongated nano-structure and method for manufacturing said apparatus**

(57)    The present invention relates to an apparatus for detecting one or more analytes, for example analytes selected from the group comprising nucleic acids, metabolites, peptides, proteins, hormones, pesticides, neurotransmitters, ions in blood, electrolytes, toxic gases, pH and biological warfare agents, the apparatus comprising an insulating substrate, at least one first electrode on the substrate at least one elongate nanostructure extending from and electrically connected to the or each said electrode and extending over the surface of the wafer away from the respective electrode, a passivating layer covering the or each electrode, but not all of said at least one elongate nanostructure, a well crossing the at least one elongate nanostructure extending from the or each electrode and forming a static reservoir for a liquid being investigated for the presence of at least one analyte, a reference electrode provided on said substrate within said well or insertable into said well and respective readout pads electrically connected to the or each electrode and to the reference electrode if the latter is provided on the substrate, the at least one elongate nanostructure being capable of being functionalized for detecting one or more analytes.

Fig. 9

EP 2 434 278 A1

**Description**

The present invention relates to an apparatus and to a method of fabricating an apparatus for detecting one or more analytes, for example analytes selected from the group comprising nucleic acids, metabolites, peptides, proteins, and hormones, the apparatus comprising an insulating substrate, at least one first electrode on the substrate at least one elongate nanostructure extending from and electrically connected to the or each said electrode and extending over the surface of the wafer away from the respective electrode.

An apparatus of this general kind rand realized as an FET is known from the document "Label-Free DNA Biosensors Based on Functionalized Carbon Nanotube Field Effect Transistors by Maria Teresa Martinez, Yu-Chih Tseng, Neres Ormategui, Iraida Loinaz, Ramon Eritja and Jeffrey Bokor published in Nano Letters 2009, Vol. 9, No.2, pages 530 to 536. As described there the possibility of label-free electrical detection of DNA-hybridization offers a new approach for a new generation of DNA chips with direct electrical readout for a fast simple and inexpensive analysis of nucleic acid samples. The detector described there utilizes FETs based on semiconductor carbon nanotubes. Such FETs are described in US patent 7,091,096 with particular emphasis being placed there on converting the unwanted metallically conducting carbon nanotubes into insulating nanotubes leaving the desired semiconducting nanotubes extending between the source and drain electrodes.

In the proposal of the above named paper by Martinez et al the detector comprises a p**Si substrate with an array of FETs. The substrate is covered with a layer of $SiO_2$ on which pairs of source and drain electrodes are formed with a carbon nanotube extending between the pairs of source and drain electrodes. The highly p-type substrate serves as a back gate for the so formed FETs. The carbon nanotubes are functionalized to detect the desired species of DNA. The document concerned is just one of many similar proposals.

Usually detectors used for detecting DNA require the DNA to be provided with some form of label. Thus conventional methods use the so called polymerase chain reaction (PCR) to amplify number of copies of DNA and to increase the sensitivity of the measurement. However, if a sufficiently sensitive detection method is available the use of PCR would be unnecessary, leading to shorter response times and less processing steps to obtain the result. There the limit of detection is at the pM level (picomol = $10^{-12}$M-without the use of PCR). The best shown sensitivity to date with any nanomaterial is about 10fM (i.e. 10 femtomol = $10^{-14}$M) using Si nanowires.

It should be noted that the present invention is not restricted to DNA as an analyte but can be used quite generally with a whole range of analytes, for example analytes selected from the group comprising nucleic acids, metabolites, peptides, proteins, hormones, pesticides, neurotransmitters, electrolytes, toxic gases, pH and biological warfare agents.

The problem with all existing detectors is that their sensitivity still leaves scope for improvement. Thus, the present invention aims at achieving sensitivities in the sub-femtomolar range as low as 100 attomol (i.e. $10^{-16}$M) or possibly better. Furthermore, it is the object of the present invention to provide apparatus of the initially named kind which, despite being highly sensitive can be manufactured economically and which represents a sound realization of the laboratory on a chip concept.

In order to satisfy these objects there is provided, in its simplest form, an apparatus of the initially named kind with a passivating layer covering the or each electrode, but not all of said at least one elongate nanostructure, , a well crossing the at least one elongate nanostructure extending from the or each electrode and forming a static reservoir for a liquid being investigated for the presence of at least one analyte, a reference electrode provided on said substrate within said well or insertable into said well and respective readout pads electrically connected to the or each electrode and to the reference electrode if the latter is provided on the substrate, the at least one elongate nanostructure being capable of being functionalized for detecting one or more analytes.

In a preferred embodiment at least one layer of insulating material overlies the or each electrode and at least part of the substrate and said well is provided in said insulating layer.

Through the provision of the passivating layer it can be ensured that the functionalization of the elongate nanostructures does not lead to functionalization of the electrodes which has been found to represent a major source of reduction of the detection sensitivity of the apparatus. The passivation layer also eliminates background electrical current which is caused by solution electrolytes present between electrodes. By the provision of a well in the substrate or in an insulating layer a liquid droplet containing the compound(s) used for functionalizing the elongate nanostructures can be brought into contact with the nanostructure(s) and this makes it very easy for the user or for an intermediary, such as a company selling chips, to prepare substrates (chips) for detecting specific analytes or groups of analytes. Furthermore, the well also enables the analyte(s) being investigated to be applied to the chips in a droplet of liquid so that any and all analytes present in the droplet can readily enter into contact with any or all the elongate nanostructures accessible through the respective well.

The well typically has a depth in the range from 1 $\mu$m to 20 mm, preferably in the range from 10 $\mu$m to 2 mm. When the well is provided in an insulating layer then this insulating layer preferably consists of PDMS (poly(dimethylsiloxine)) which tends to have a sticky surface and therefore bonds readily to the underlying substrate and structures provided thereon. The well can be defined within this layer of insulating material and can, for example be cut out prior to the application of the insulating layer using a knife, such as a scalpel, or in automated manner by a machine. There are many alternatives to PDMS. Another possibility would be to use an insulating layer of SU-8 which is an epoxy-based negative photoresist commonly used in the field of semiconductors, obtainable from companies such as microchem and Gersteltec.

Furthermore, by providing a reference electrode on the substrate or dipping it into a liquid volume contained in the well, the reference electrode can act both as a gate and as a reference which greatly facilitates the calibration and read out from the individual FETs. This is especially useful when a plurality of elongate nanostructures are present extending away from one electrode and are all exposed to the same reactant within the well irrespective of whether they are all functionalized to detect the same analyte or are functionalized to detect different analytes.

It is a special feature of the present invention that the gate electrode is either provided on the substrate within the well or is provided as a separate electrode contacting the liquid in the well, be it a liquid used to functionalize or chemically prepare the elongate nanostructure or a liquid containing analyte. The gate electrode is preferably designed as a reference electrode such as is commonly used in electrochemistry, but not however for the purpose of detecting minute quantities of analyte. Such reference electrodes are commercially available in the form of a silver wire coated with AgCl and stored in a moist state. If the reference electrode (gate electrode) is provided on the substrate then it typically comprises a pad of silver coated with AgCl and covered with a layer of hydrogel to keep it moist. It is also possible to use other materials such as Pd or any Group VIII metal. As will be explained later when the reference electrode is used in connection with the functionalization of the elongate nanostructure(s) the potential difference between the reference electrode and the potential applied to the elongate nanostructure(s) via an associated electrode (and a pad and conductive trace) can be carefully controlled to control the electrochemistry of the functionalization. In addition, during the actual measurement, different gate voltages can be applied to the electrode to enable measurement in a number of different bias ranges.

The apparatus of the invention as described above need not be configured as a chip having a plurality of FETs thereon but can also be configured to operate with elongate nanostructures extending on a substrate away from a first electrode or from a plurality of first electrodes, as will be explained later in more detail.

A particularly preferred form of the apparatus is, however, configured as one or more FETs and further comprises at least one second electrode provided on said substrate and spaced from said first electrode by a gap, said at least one elongate nanostructure extending between and electrically connected to a first electrode and a second electrode across said gap, said passivating layer covering each of said first and second electrodes, but not all of said at least one elongate nanostructure, the at least one layer of insulating material overlying the first and second electrodes and the well crossing the at least one elongate nanostructure in the gap and forming a static reservoir for a liquid being investigated for the presence of at least one analyte, a reference electrode provided on said substrate within said window or insertable into said well and readout pads electrically connected to the first and second electrodes and optionally to the reference electrode if provided on the substrate, the at least one elongate nanostructure crossing said gap being capable of being functionalized for detecting one or more analytes.

Most preferably there are provided a plurality of pairs of first and second electrodes on the same substrate, each pair of first and second electrodes being spaced apart by a respective gap and having respective readout pads, respective elongate nanostructures crossing said gaps, the one-dimensional nanostructure or nanostructures crossing said gap being respectively functionalized for the one or more analytes, there being a common well extending over all said gaps or a respective well for each said gap or for a group of gaps.

An apparatus or a chip of this kind is particularly suited for automatic detection of analytes on an industrial scale. Each FET formed by a pair of first and second electrodes and the associated elongate nanostructure can be designed to detect a specific analyte or a plurality of different analytes if an appropriate number of suitably functionalized elongate nanostructures are provided for each pair of electrodes. When plural FETs are provided each can be designed for detecting the same analyte or plurality of analytes. Alternatively, the individual FETs or groups of them can be designed for detecting different analytes or different pluralities of analytes. The corresponding substrate or chip bearing the array of FETs can be positioned beneath an automatic dispenser for samples for investigation, a so-called spotter, and the spotter can be operated to dispense a drop of the sample or samples into the respective wells. This process can take place under computer control so that there is a unique association between each sample and the FETs on the chip and an equally unique and unambiguous association between the detector read-outs and the individual FETs. Furthermore, the reference electrode or electrode facilitates rapid and unambiguous calibration of the apparatus and of the detection result.

The apparatus described above is particularly preferably used in combination with readout circuitry connectable or connected to the respective readout pads and to said reference electrode and adapted to carry out an AC measurement of complex impedance, i.e. of magnitude and phase, for the or each pair of first and second electrodes respectively.

Whereas, in the prior art, the read-out of the impedance values has been realized as a DC read-out, it has been found that a measurement of the complex resistance, i.e. with respect to both magnitude and phase, leads to a much higher sensitivity which contributes significantly to the desired sensitivity of detection of analytes, i.e. makes it possible to reliably detect even lower concentrations of analytes.

There is no particular restriction on the type of elongate nanostructure that is used and thus said one-dimensional nanostructure or nanostructures can be selected from the group comprising single-wall carbon nanotubes with metallic and/or semiconducting character, carbon nanowires or a graphene nanoribbon, metallic nanowires, for example of gold or palladium or platinum, polymer nanowires, for example polyaniline (PANI) or polypropylene vinylene (PPV) which have metallic or semiconducting character and inorganic nanowires, such as ZnO, or a combination of any of the above. The nanostructures preferably have a major cross-sectional dimension of not more than 50 nm and especially preferably of not more than 10 nm and most especially preferred of 5 nm or less. Carbon nanotubes typically have a diameter of 1 nm and bundles of carbon nanotubes have diameters of typically 3 to 4 nm. The elongate nanostructures are preferably one-dimensional nanostructures which are understood in the art and for the purpose of this description to mean nanostructures having a ratio of length to the major cross-sectional dimension of at least 10:1 1 and preferably of more than 50:1, for example 100:1.

The layer of insulating material preferably comprises a layer of a material selected from the group comprising: PDMS, polyimide, polyethylene, SU8, glass, $SiO_2$ $SiO_x$, or $S_{13}N_4$.

The substrate is preferably a material selected from the group comprising glass, polyimide, silicon/silicon oxide, quartz and silicon nitride.

In a preferred embodiment the apparatus is provided in combination with an optical excitation source such as a laser for the optical excitation of a substance present in the well. The optical excitation source is preferably selected to generate surface plasmons in the substance. In this case an optical coupling device can also be provided at the apparatus, for example, at the rear of the substrate for coupling optical signals into the well in the vicinity of the substance.

This ensures that the electrical detection of e.g. non-charged species present in the well can be augmented with a source of optical excitation, preferably using a laser whose variable parameters include the wavelength, the intensity, the angle of illumination and the modulation of intensity and / or wavelength. The laser excitation can be coupled to the substrate with or without a prism in order to generate surface plasmons. The mechanism involves but is not restricted to photoconductivity, photothermal effects, photovoltage / photocurrent generation, photodesorption, plasmons etc. The measured magnitude and phase photo-Z-maps, which are a function of the signal frequency, gate voltage, laser amplitude, laser wavelength, illumination angle and laser modulation constitute the sensor signal.

The present invention further relates to a method of fabricating an apparatus for detecting one or more analytes, especially an apparatus as claimed in any one of the preceding claims, the method comprising the steps of:

a) fabrication of electrodes on a substrate,
b) deposition, growth or transfer of at least one elongate nanostructure on the substrate with one end contacting a respective electrode, with step b) being carried out before or af ter step a)
c) deposition of a passivating layer over the or each electrode contacted by the one-dimensional nanostructures,
d) forming a well therein extending across said one-dimensional nanostructure or nanostructures either in the substrate or in an insulting layer over the electrodes and the substrate
e) providing a respective readout pad connecting to each said at least one electrode and/or
f) electrochemically treating said nanostructures and/or
g) functionalizing said nanostructures with a receptor or receptors specific to an analyte

It will be noted that feature b) speaks of deposition, growing or transfer of the at least one nanostructure. In this case the electrode(s) can be formed after deposition of the elongate nanostructure(s), for example by a printing technique or by a lithographic technique or by a PVD e.g. a sputtering technique, or by a CVD technique or galvanically.

In this connection deposition will be understood to mean that the elongate nanostructures are separately formed and subsequently deposited on the substrate. This can be done, together with a certain degree of alignment of the elongate nanostructure(s) by dielectrophoresis and in this case the electrode(s) to which the elongate nanostructure(s) is/are secured at at least one end are previously deposited on the substrate and are used to carry dielectrophoresis. Alternatively, the surface of the substrate can be structured, for example by lithography to form channels into which the elongate nanostructure(s) are preferentially deposited so that they are generally aligned into the channels. In this case the electrode(s) can be formed after deposition of the elongate nanostructure(s), for example by a printing technique or by a lithographic technique or by a PVD e.g. a sputtering technique, or by a CVD technique or galvanically.

Alternatively a self-organized surface layer can be used. For example it is known that epitaxial silicon can be grown with a corrugated surface, with about 3 nm amplitude at a suitable growth temperature. Such a surface could subsequently be oxidized and the corrugations used to align elongate nanostructures.

It is also possible to transfer the elongate nanostructures e.g. in the form of graphene ribbons from a carrier to the surface of a substrate, again prior or after the formation of the respective electrodes.

The method preferably comprises fabrication of at least one first electrode and at least one second electrode on said substrate, said at least one second electrode being spaced from said first electrode by a gap, and deposition or growth of at least one elongate nanostructure such that the or each elongate nanostructure has a first end contacting said first electrode and a second end contacting said second electrode, and forming said well across said gap.

It is particularly convenient when the method also includes the step of forming a reference electrode on said substrate at a position such that it lies within said well.

The method also preferably includes the step of forming a plurality of pairs of first and second electrodes on the same substrate, each pair of first and second electrodes being spaced apart by a respective gap and having respective readout pads, and forming a plurality of elongate nanostructures crossing each said gap, and functionalizing the plurality of elongate nanostructures crossing said gap for the one or more analytes, and providing in said insulating layer either a common well extending over all said gaps or a respective well for each said gap or for a group of gaps.

Further preferred embodiments of the method are set forth in the subordinate claims.

The invention will now be explained in more detail with reference to embodiments as shown in and described by way of example only with reference to the accompanying drawings in which:

Figs. 1A- 1I          show diagrams to illustrate the steps in the manufacture of a detector in accordance with the present invention,

Fig. 2                shows a plan view of an apparatus in accordance with the present invention having two separate detectors on one chip, each having three first electrodes, three second electrodes and one reference electrode,

Fig. 3                shows a plan view of an alternative pair of detectors in accordance with the present invention, with the detectors each having three first electrodes and one reference electrode but no second electrodes,

Figs. 4A, 4B          show diagrams to explain the dielectrophoretic deposition of carbon nanotubes in contact with an electrode of a detector,

Figs. 5A to 5B        show preferred ways of functionalizing the single-wall carbon nanotubes preferably used as elongate nanostructures.

Figs. 6A, 6B          are circuit diagrams illustrating the operation of a frequency analyzer used in accordance with the present invention,

Figs. 7A, 7B          are typical three-dimensional impedance maps obtained using the frequency analyzer explained with reference to Figs. 6A and 6B,

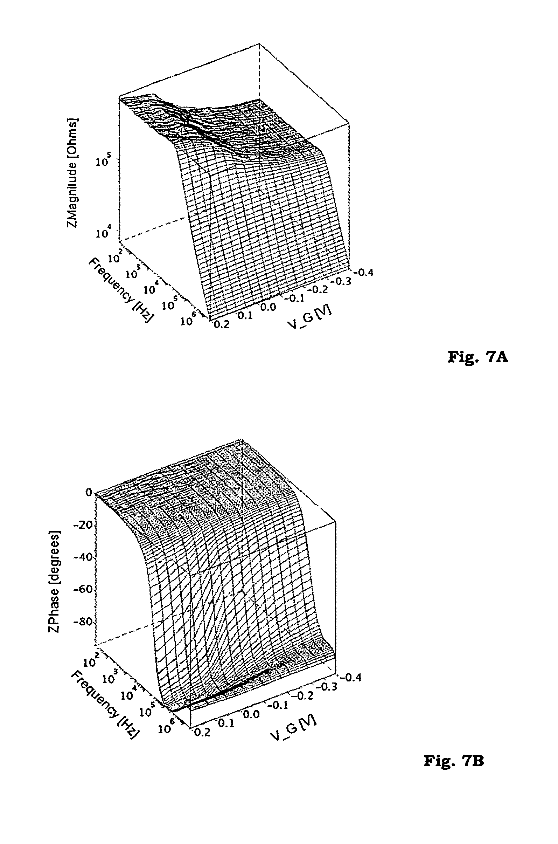

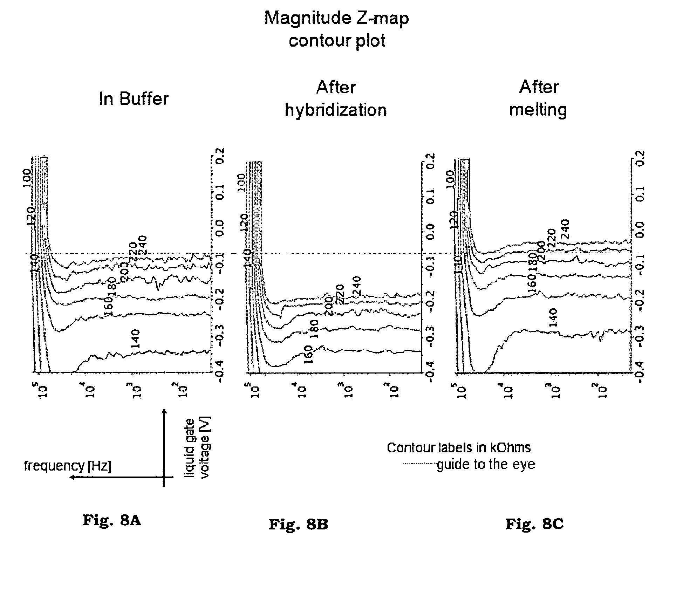

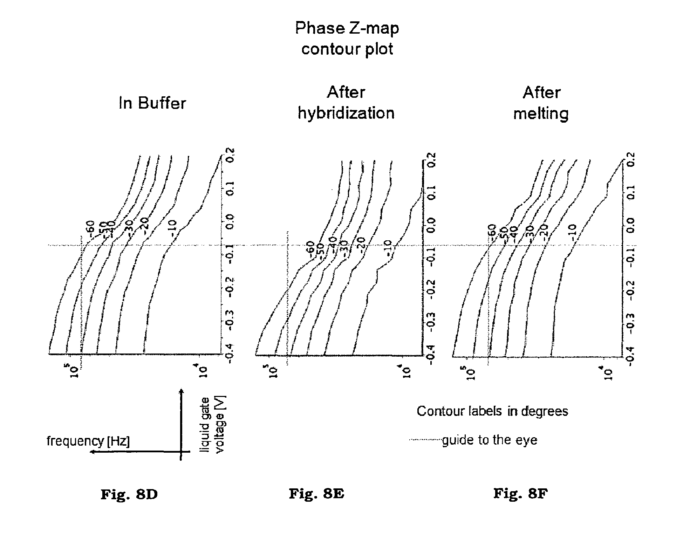

Figs. 8A-8F           are diagrams equivalent to Figs. 7A and 7B but in two dimensions with Figs. 8A to 8C showing magnitude in kOhm of the complex impedance as a function of frequency for different applied gate voltages measured for three cases, namely with Fig. 8A showing the measured magnitude for the functionalized single-wall carbon nanotube(s) in a buffer solution, Fig. 8B showing the measured magnitude for the same carbon nanotube(s) after addition of the analyte solution containing the DNA solution after hybridization, Fig. 8C shows the measured magnitude after melting, i.e. removal of the DNA by washing the detector with water, again measured in the buffer solution (without analyte), Figs. 8D to 8F show the phase of the complex impedance in degrees as a function of the applied frequency for different gate voltages, with Fig. 8D showing the phase angle of the measured impedance in the buffer solution, Fig. 8E showing the phase angle of the measured impedance after the addition of the analyte and hybridization, and Fig. 8F showing the phase angle "after melting",

Fig. 9                shows a schematic representation of a detector in accordance with the invention,

Figs. 10A to 10C      show AFM images of various detectors in accordance with the invention,

Fig. 10D              shows a graph showing the detector response of the detectors shown in Figs. 10A to 10C, and

Figs. 11A & 11B       show a possible alternative realization of the invention using optical detection.

General description of detector topographies and their manufacture

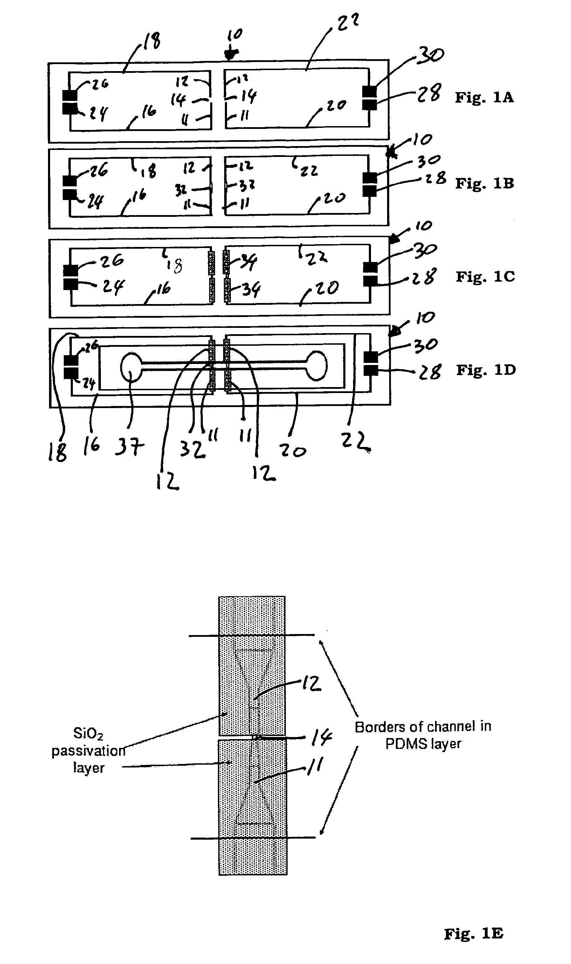

Turning now to Fig. 1A there can be seen a substrate 10 which can, for example, consists of a material selected from the group comprising glass, polyimide, silicon/silicon oxide, quartz and silicon nitride. In the present case the substrate 10 consists of an insulating silicon dioxide layer of 500 nm thickness which is an insulator on an $n^+$-type substrate of crystalline Si with a resistivity of 1 Ohm/cm$^2$ and of 500 $\mu$m thickness.

It should be noted that references in the specification to silicon oxide Means $SiO_x$, where x can have values between 1 and 2 and signifies a non-stoichiometric oxide. However, $SiO_2$ i.e. Silicon dioxide is a perfectly viable and extremely useful silicon oxide for the purposes of the present invention both, as an insulating layer on a conductive silicon substrate and as a passivation layer.

Two pairs of first and second electrodes 11 and 12 of, in this case, platinum are deposited on the substrate 10 and one pair of oppositely disposed electrodes 11, 12 is shown to an enlarged scale in Fig. 1E. The electrode need not necessarily be made of platinum; they could for example consist of Au or Pd or any other metal or conducting material. One notes that the first and second electrodes 11, 12 of each pair are spaced apart by a gap 14, typically of about 40 $\mu$m. Each electrode 11, 12 is connected via a respective conductive trace 16, 18, 20, 22 to a respective readout pad 24, 26, 28, 30.

The traces 16, 18, 20 and 22 and the readout pads 24, 26, 28 and 30 are all preferably made of the same material as the electrodes as these can then be deposited in the same method step and they are typically deposited on the substrate by a lithographic technique, as are the first and second electrodes.

It should be noted that in this example just two first electrodes and two second electrodes are shown on the same substrate. In actual practice a significant number of such first and second electrodes will be provided on one substrate, for example the substrate could be divided into a number of chips each having first and second pairs of electrodes, so that one substrate could yield a plurality of individual chips.

There is also no restriction to combining first and second pairs of first and second electrodes adjacent one another on a substrate, instead there could be just a single pair of first and second electrodes or a larger number of pairs of first and second electrodes disposed adjacent to one another on each sub-area of a larger substrate. Also, once the manufacture of the detectors on the large area substrate is complete, the sub-area can be separated physically from one another for use by appropriately cutting up the substrate, or the substrate with the matrix of detectors can be used for sample analysis in an automated large scale system.

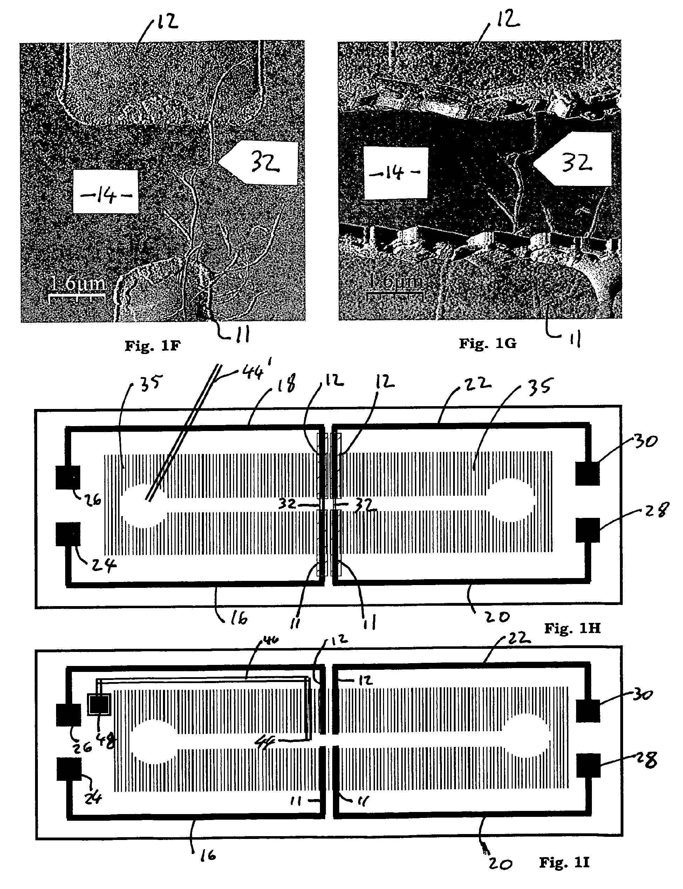

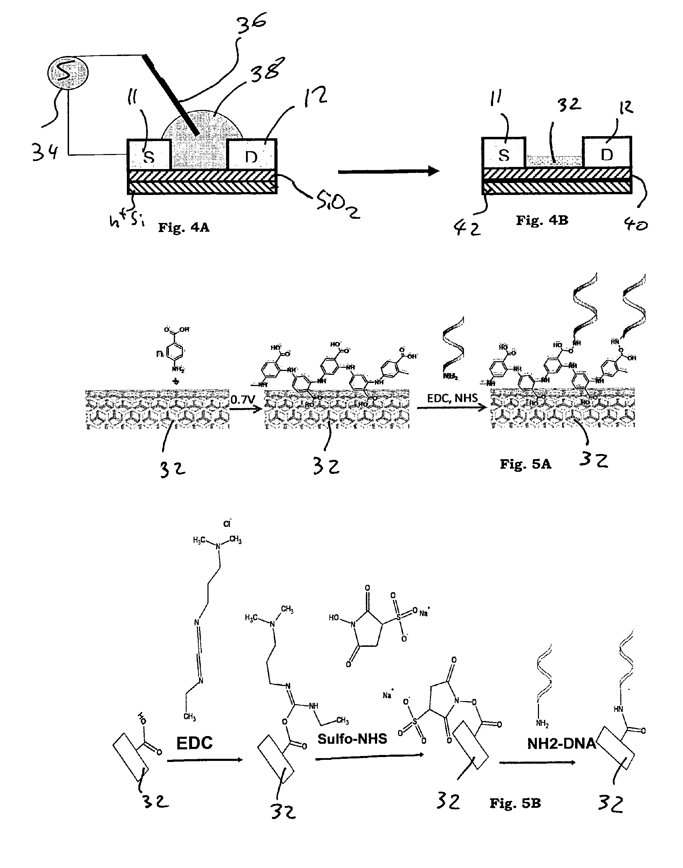

The next step in the preparation of the detector is to deposit elongate nanostructures so that these nanostructures contact the first electrode 11 at one end and the second electrode 12 at the other end. This is schematically shown in Fig. 1B where elongate nanostructures in the form of single-wall carbon nanotubes (CNTs) 32 have been drawn in extending across the gaps 14 between the pairs of first and second electrodes 11, 12 in Fig. 1B. A typical example of how a carbon nanotube can be visualized crossing the gap 14 is shown by the atomic force microscope pictures of Figs. 1F and 1G which show in Fig. 1F a single CNT dielectrophoretically trapped across the gap 14 between the two first and second electrodes 11, 12. Dielectrophoretic alignment/ deposition of carbon nanotubes will be explained subsequently with reference to Figs. 4A and 4B.

Fig. 1G shows the same situation as Fig. 1F but after the pairs of first and second electrodes 11, 12 have been passivated by the silicon oxide layer 34 provided on them as shown in Fig. 1C. The passivation step will be described later in more detail.

After the passivation of the electrodes a dielectric/insulating layer 35 is provided crossing the central region of the chip as shown in Fig. 1D and in this insulating layer 35 consists of a material selected from the group comprising PDMS, polyimide, polyethylene, SU8, glass, $SiO_2$ $SiO_x$ or $Si_3N_4$ and is preferably PDMS. In the insulating layer 35 there is formed a well 37 which is generally of elongate dumb-bell shape, with the channel between the two generally circular ends of the well 37 standing transverse to the single-wall carbon nanotubes 32 and generally being of a width corresponding to slightly less than the separation between the first and second electrodes 11 and 12. Once the well has been formed, the device is basically complete apart from the reference electrode that is used.

There are two ways of achieving this. As shown in Fig. 1G, the reference electrode 44 can be formed simultaneously with the first and second electrodes 11 and 12 on the substrate is positioned in the channel of the well 37 and therefore exposed to any liquid present in the well 37. In this case the reference electrode 44 is connected via a respective trace 46 to a respective contact pad 48 so that bias voltages or measurement voltages can be applied to the reference electrode as required. The other alternative, which is illustrated in Fig. 1H, is to simply use an external electrode 44' which is not provided on the substrate but rather contacts the liquid provided in use in the well 37 and which can, for example, be realized as a silver wire. The reference electrode 44 in Fig. 1J is also typically of silver coated with Agcl. Both reference electrodes 44 and 44' could, however, be of other materials, such as Pt or a saturated calomel electrode.

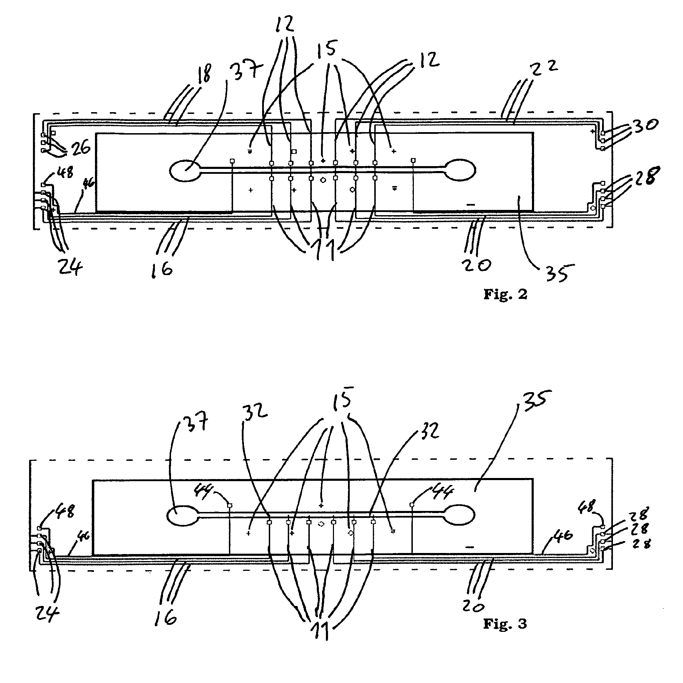

Fig. 2 now shows an arrangement which is not dissimilar to that of Figs. 1A to 1J, but in which two separate detectors 10, each having three first electrodes 11 and three second electrodes 12, are provided on a common chip. In Fig. 2 the same reference numerals have been used as in Figs. 1A to 1J (a convention which will be used throughout this description) and have the same meaning or at least identify the same function as has been described in connection with Figs. 1A to 1J. For this reason the parts will not be described further here unless there is something additional of merit to discuss.

Also shown in Fig. 2 and also present in the same way as in the embodiment of Fig. 1D is the insulating layer 35 with the well 37. Again it can be seen that there is a channel of the well 37 which extends generally transverse to the elongate nanostructures 32 and the central portions of the elongate nanostructures 32 are exposed to a liquid introduced in use into the well 37 where it forms a static reservoir. Alignment marks 15 can be provided on the substrate 10 prior to the application of the remaining detector components, to ensure that the respective detector components are correctly aligned on the substrate 10.

It should be noted that Fig. 2 is bounded at the top and the bottom by dotted lines. This simply signifies that the diagram of Fig. 2 is just one sub-area of a larger substrate with further similar (or dissimilar) detectors being provided on the same substrate both above and below the sub-area shown in Fig. 2.

A discussion will now be given of the embodiment of Fig. 3 which differs from the embodiment of Figs. 1A-1J in that it is not realized as an FET device with the elongate nanostructures extending between source and drain electrodes.

What is shown in Fig. 3 is an area of a substrate which has been lithographically patterned to form two separate detectors each having three first electrodes 11 and respective reference electrodes 44. As in Fig. 1J, each reference electrode 44 is connected by a conductive trace 46 to a respective pad 48. Likewise, each first electrode 11 is connected by a respective conductive trace 16 to a respective contact pad 24. Likewise, the electrodes 11 of the detector at the left-hand side of the drawing are connected via respective traces 16 to respective readout pads 24. The first electrodes 11 of the three electrodes at the right-hand side of Fig. 3 are again connected via respective traces 20 to respective readout pads 28.

The dielectrophoresis technique described with reference to Figs. 4A and 4B can also be used to attach single or multiple single-wall carbon nanotubes 32 to respective electrodes 11 in Fig. 3 so that the carbon nanotubes extend generally radially away from the first electrodes 11. To achieve this, the drop of liquid containing the single-wall carbon nanotubes is placed over the electrodes 11 and the alternating source 34 is connected to each electrode 11. Following this a current can be passed to the drop of liquid via the electrode 11 to cause the desired alignment of the carbon nanotubes 32 present in the drop of liquid.

Some important aspects of the present invention will now be discussed in more detail:

Dielectrophoretic deposition of elongate nanostructures

The preferred way of depositing the carbon nanotubes between each pair of first and second electrodes 11, 12 is by way of dielectrophoresis. The technique is illustrated in Figs. 4A and 4B.

The invention can basically be used with any form of single-wall carbon nanotube and hitherto experiments have been carried out with single-wall carbon nanotubes produced by the HiPCO process (High Pressure Pyrolysis of Carbon Monoxide) as performed by Tubes@Rice, Lexington, USA. Such single-wall carbon nanotubes can be dispersed in a surfactant solution (typically Triton X-100, SDS, SDBS or other) while using ultrasonic agitation to ensure the single-wall carbon nanotubes in the sample are distributed throughout the surfactant solution. A drop of the surfactant solution is then placed on the chip as prepared in Fig. 1A so that it covers the gaps 14 between the pairs of first and second electrodes 11 and 12. A source of alternating voltage 34 is then connected to the source electrode at one end and to a platinum wire 36 at the other terminal, with the end of the platinum wire 36 dipping into the drop of the solution containing a few single-wall carbon nanotubes 38.

An effective alternating field generated in the drop 38 causes one or more single-wall carbon nanotubes to extend between the source and the drain electrodes 11 and 12, with one representative carbon nanotube being identified by 32 in Fig. 4B. The cross-sectional diagrams of Figs. 4A and 4B also show the makeup of the substrate on which the source and drain electrodes are positioned. This comprises a layer of amorphous $SiO_2$ 40 which is an oxide thermally grown on an $n^+Si$ crystalline substrate 42.

In accordance with the invention it has been found that dielectrophoresis can be carried out with a very much improved trapping effect by applying the AC voltage used to trap carbon nanotubes, or other elongate nanostructures between just one electrode and a conductive wire (e.g. Pt) dipping into the surfactant solution containing the carbon nanotubes or other nanostructures. In this scenario, the electromagnetic field is concentrated at the electrical double layer at the interface between the electrode and the solution. This should be contrasted with prior art dielectrophoresis methods which use an AC voltage applied between the first electrode and a second electrode on or below the substrate. The advantage of the present method is that the nanotubes can be simultaneously deposited at a plurality of electrode gap positions without the need for a second electrode being present on or below the substrate. Using this method e.g. carbon nanotubes 32 have been successfully simultaneously deposited at 60 positions on a glass wafer (such as the wafer shown in Fig.3), this was previously not possible using the prior art methods.

Passivation

The passivating layer 34 shown in Figs. 1C and 1G and present in all embodiments can be realized in a variety of different ways and materials. In the preferred arrangement the passivating layer 34, which in the embodiment of Fig. 1C simultaneously covers both first electrodes 11 and both second electrodes 12, consists of silicon oxide. The silicon dioxide passivation layer is deposited by doing a second lithography step, which is aligned to the electrode positions on the wafer. At the end, all of the electrode lines up to the position of the gap are covered with silicon oxide. Only the surface of the nanotube will be subsequently in contact with the liquid during the sensor trials. Possible alternatives for passivation are: polymer resins which act as a barrier, PDMS, SU-8 and electrically nonconducting chemically non-reactive materials. Electroless deposition e.g. of Ni on gold and subsequent oxide formation can also be used as an alternative route for the passivation of electrodes.

The AFM image of Fig. 1G which was taken after the passivation has been completed shows how the carbon nanotube 32 extending between the first and second electrodes 11, 12 is also covered with the passivating layer at its junction to the boundaries of the first and second electrodes 11, 12. This passivating layer has been found to be particularly advantageous. If it is not provided then the functionalization of the elongate nanostructure crossing between the electrodes 11 and 12 also covers parts of the first and second electrodes 11, 12 and this leads to faulty detection signals which actually increase the amplitude of unwanted noise relative to the wanted signal strength from the carbon nanotube. In the case of non-passivated electrodes the background current flowing through the analyte solution could affect the sensing results.

Functionalization

As mentioned above, detectors of the general kind discussed can be used to detect a whole variety of analytes, such as nucleic acids, metabolytes, peptides, proteins and hormones. The functionalization scheme preferred for use for the present invention can be summarized as follows with reference to Figs. 5A and 5B:

It should be noted that this functionalization can be carried out once the elongate nanostructure(s) has/have been deposited, grown or transferred to the substrate, but can also be carried out before the elongate nanostructure(s) has/have been deposited or transferred to the substrate. Particularly preferred, however is the functionalization of the elongate nanostructure(s) after it/they has/have been deposited, grown or transferred to the substrate, and it is a particular advantage of this application that the functionalization or at least the first step is carried out in an electrochemical method in a liquid present in the well and carried out using the reference or gate electrode.

In Fig. 5A the reference numeral 32 again points to a single-wall carbon nanotube (SWCNT) and the generally hexagonal carbon structure of the surface of a carbon nanotube is schematically illustrated. By applying a positive voltage of 0.7 V to the SWCNTs with ethanol solution of 5 mM aminobenzoic acid (ABA), 100 mM $LiC10_4$ introduced into the well 37 the aminobenzoic acid is oxidized and polymerized. This potential difference is applied to one of the first and second electrodes and the reference electrode. This results in a polymer coating with a thickness of 2-3 nm around the nanotubes. This results in dangling COOH bonds at the surface of the polymer which, in a second functionalization step can be bonded to a receptor specific to the analyte to be detected. Such receptors will be discussed later with reference to specific embodiments. For example, due to the formation of polymer around the tubes $NH_2$-DNA was attached to the COOH groups of the polymer using the EDC/NHS coupling process.

Carbodiimide coupling is widely used for the formation of -CO-NH- (peptide) bonds using carboxyl (COOH)- and amino (NH2)- groups of components to be conjugated. In the presence of *N*-hydroxysulfosuccinimide (Sulfo-NHS) (see Fig. 5B), 1-Ethyl-3-[3-dimethylaminopropyl]carbodiimide hydrochloride (EDC or EDAC) is used to convert COOH-groups to amine-reactive Sulfo-NHS esters. This is done by mixing the EDC with a carboxyl containing molecule and adding Sulfo-NHS. Formation of stable -CO-NH-bond occurs after adding molecules containing amino-groups.

The functionalization procedure to attach the probe sequence is shown in Fig 5A. First, 4-aminobenzoic acid (ABA) is electropolymerized on to the nanotube surface. This results in the non-covalent wrapping of the nanotubes with -COOH groups. In a second step, 5'-$NH_2$-DNA was coupled to the carboxylic groups using carbodiimide chemistry with ethylenediamine chloride (EDC) and N hydroxysuccinimide (NHS). I.e. in the second step amino-functionalized probe DNA is covalently coupled to the COOH groups by carbodiimide chemistry. The non-functionalized sections are subsequently blocked with ethanolamine. The height increase upon electropolymerization was estimated to be around 2-3 nm as obtained from AFM images. The electrochemical functionalization protocol is unique and offers a number of key advantages. First, the functionalization is site-specific, i.e. only the nanotubes addressed by the electrochemical modification are preferentially decorated with probe DNA. This also ensures that there is no DNA in the vicinity of the nanotube on the chip surface.

Because the polymer layer is applied by electrochemistry the possibility now exists of addressing each nanostructure or group of nanostructures in the well separately by applying the potential difference to the reference electrode, on the one hand, and to sequential nanostructures or groups of nanostructures, on the other hand. Provided a receptor specific to a specific analyte is applied to each electrochemically addressed nanostructure or group of nanostructures in turn, then each nanostructure or group of nanostructures communicating with the same well can be provided with a different receptor (or the same receptor if multiple measurements are deemed useful to obtain better average values). If the receptors for specific analytes are also susceptible to being electrochemically deposited then it may be possible to functionalize all nanostructures crossing the well with a common polymer and to sequentially add the receptors to individual nanostructure or groups of nanostructure by changing the liquid containing the receptor in the well and electrochemically addressing the individual nanostructures or groups of nanostructures in turn. Alternatively it may be necessary to carry out the first step of functionalization with the e.g. ABA (or other suitable materials) for the first nanostructure or group of nanostructures, to then flush the remainder of the liquid from the well, to then carry out the second step of functionalization, i.e. the attachment of the relevant receptor to the dangling bonds to then flush this material from the well and replace it with further material for the first functionalization step. Then the second elongate nanostructure of group of nanostructures is/are addressed electrochemically and this process is repeated until all nanostructures or groups of nanostructures are suitably functionalized (first and second step functionalization).

In many cases if a plurality of like detectors is being made then all detectors on a common substrate can be functionalized together (i.e. first and second step) and then individual sensors can be separated from the wafer, or the wafer can be used as a whole in a batch processing plant (such as a spotter).

From AFM images, the height of the polymer layer is estimated to be around 1 to 3 nm. On the one hand, this layer ensures that the oligonucleotides do not wrap around the nanotube. On the other hand, the high density of COOH groups on the CNT surface ensures an efficient coupling of probe sequences via standard carbodiimide chemistry. Furthermore, this ensures that the probe sequences are preferentially immobilized only on the nanotube surface. Unfunctionalized areas are subsequently blocked with ethanolamine. By blocking the unfunctionalized areas with ethanolamine, the nitrogen ions in the ethanolamine react with the hydrogen ions of the remaining carboxyl group to form a salt, the well can subsequently also be treated using a phosphate buffer, such as the buffer solution mentioned below, like the ethanolamine the phosphate buffer is also an alkali substance which combines with the acid remaining in the well and around the nanotubes to form a salt. The blocking process effectively counteracts any remaining acid present in the well by combining with the acid to produce a salt. This has the effect that any solution which is subsequently added to the well will not react with the acid and the properties of the subsequently added solution will not be destroyed by the acid.

It is noted that it was extremely difficult to provide a covalent bond to a carbon nanotube, for example very high temperatures and/or the use of strong acids to "cook" the nanotube(s) were required to form such covalent bonds. However, in accordance with the present invention electrochemistry, which is much easier to do, provides a facile way to provide a covalent bond on the carbon nanotube, as it does not destroy the nanotube or its environment and is a far more stable and reproducible method. The covalent bonds have the effect of changing the resistance of the nanotube considerably, i.e. increase the resistance significantly.

In one aspect of the present invention, the use of electrochemistry advantageously also allows the shift to non-covalent bonds. The advantage is that the functionalization is restricted to only the nanotubes. Depending on the required situation one can select either covalent or non-covalent bonds. Using amino functionalized or diazonium functionalized receptors, the nanotubes can be respectively modified non-covalently or covalently.

Examples of functionalization for different analytes:

a) Metabolite, e.g. glucose here a synthetic receptor, boronic acid, is attached either covalently or non-covalently to detect the glucose.

b) Peptides, e.g. amyloid beta peptide (Aβ) (Alzheimers disease), an Aβ antibody is attached to the COOH or to an aptamer for Aβ.

c) Proteins, e.g. thrombin, here a thrombin aptamer is attached and used as a receptor.

d) Hormones, e.g. insulin, an anti-insulin antibody is used as the receptor.

e) Pesticides and nerve agents, one can use a Zirconia containing compound as the receptor (Electrochemical Sensor for Organophosphate Pesticides and Nerve Agents Using Zirconia Nanoparticles as Selective Sorbents. Guodong Liu and, Yuehe Lin. Analytical Chemistry 2005 77 (18), 5894-5901)).

f) Electrolytes e.g. sodium, potassium or cesium ions, one can use specific crown ethers through direct attachment using electrochemistry.

g) Gases, e.g. hydrogen, one can deposit Pd electrochemically from $Na_2PdCl_4$, as a direct deposit.

h) Gases, e.g. H2, here Pd is used as a catalytic receptor.

i) Biological warfare, e.g. Anthrax, anti-B. anthracis antibodies or specific DNA sequences are used as a receptor.

The sensor response of the fabricated detectors to 1fM of target DNA in buffer is shown in Figs. 8A to 8F and will be discussed later in more detail. Generally speaking the sensing trials were performed for a plurality of devices using varying concentrations of DNA in 10 mM potassium phosphate buffer with 0.1 M NaCl (pH 7.4).

The impedance characteristics are first obtained in a 10 mM potassium phosphate buffer solution containing 0.1 M NaCl pH 7.4 without the target DNA. The resistances of the devices investigated are of the order of 100 kOhm which is dominant at low frequencies. It should be noted that the actual resistance arises not only in dependence on the SWCNT(s) that is/are actually connected to the first and second electrodes but also on the number of attachment to any one pair of electrodes which typically lies in the range from 1 to 10. Resistances in the range from 30 to 1000 kOhm are certainly useful.

At high frequencies capacitive components arising both from the electrical double layer formed by the conduction electrode and the ions attached thereto and the substrate are dominating as can be seen from Fig. 8A. This is particularly apparent in the corresponding ZPhase map of Fig. 8D, where it is shown that at high frequencies the phase of the impedance becomes -90°. Upon introduction of DNA into the microwell formed by the well (DNA present in a 10mM potassium phosphate buffer solution having a pH of 7.4 and containing 0.1M NaC1), the device shows a clearly different response (Fig. 8B). It is apparent that the Z Magnitude has increased and the phase has become more capacitive. After subsequent "melting" of the double strand at 80°C and washing in hot water at 80°, the initial impedance of the sensor is recovered (Fig. 8E and 8F).

Experiments with nanotubes that were not functionalized with ABA and the subsequent coupling of probe DNA did not show any significant response towards complementary sequence even at the highest concentration used in the experiments to date, i.e. 1 pM. Confirmation for the attachment of probe DNA to the functionalized surface has been obtained by using complementary sequences attached to 60 nm gold nanoparticles. The gold nanoparticles were not observed when the nanotube was not functionalized with polyABA and the probe sequence. To gather proof for the fact that the complementary sequence hybridizes indeed under the conditions used here, biotin modified DNA sequences were allowed to hybridize during the gate sweep. Subsequently, streptavidin gold was deployed to confirm the hybridization. These positive and negative control experiments showed the efficiency of the detector of the present invention.

Other possible functionalization schemes have been described in the prior art literature and could be used in place of the functionalization scheme described above.

It should be appreciated that many other possible functionalization schemes have been described in the literature, i.e. are known per se, and can also be used with the detectors of the present invention. However, these other functionalization processes do not involve the use of electrochemistry. For example the article by Martinez et al referred to above discusses the use of a CNT field effect transistor for the electronic detection of biotin-streptavidin, human immunoglobulin (IgG), various monoclonal antibodies and pig serum albumin. The same paper also discusses the detection of DNA hybridization. The paper presents a methodology for avoiding non-specific DNA absorption on CNTs and providing at the same time a stable binding for DNA probes through robust amide linkages. In the method described there the DNA probes are covalently bonded to a polymer that is anchored non-covalently to the CNT walls. More specifically, poly(methylmethacrylates$_{0.6}$-co-polyethyleneglycolmethacrylate$_{0.15}$-co-N-succinimidyl methacrylate$_{0.25}$) was designed to provide a bond between CNTs and DNA and simultaneously to prevent any other non-specific absorption.

Another review article in this field is "Carbon nanotube field-effect transistor-based biosensors" by Brett Lee Allen, Padmarka D. Kichambare and Alexander Star published in Advanced Materials 2007, 19, 1439-1451. That review article describes the use of nanowire field effect transistors for direct electrical detection of biomolecules and mentions detection of proteins, DNA hybrids and carbohydrates as well as cancer markers. It is stated that functionalization can be achieved by covalent and non-covalent modification. For example covalent modification is said to involve oxidation of carbon nanotubes to form free carboxyl groups which undergo coupling with amino groups in proteins. Non-covalent modification is said to involve bi-functional molecules as exhibit $\pi$-$\pi$ stacking on the sidewalls of the carbon nanotubes such as can be achieved by the use of a pyrene moiety commonly used for graphite functionalization.

The same article also discusses antibody-antigen interaction and says that specific sensitivity can be achieved by employing a recognition layer that induces chemical reactions and modifies the transfer characteristics. In this connection the paper discusses devices made sensitive to streptavidin by using individual biotin functionalized carbon nanotube arrays bridging two microelectrodes. Also discussed is the coating of a mixture of two polymers (poly(ethyleneimmine) and poly(ethyleneglycol). The former provides amino groups for the coupling of biotin-N-hydroxy succinimidyl ester and the latter prevents the non-specific absorption of proteins on the functionalized carbon nanotubes. The same paper also discusses the proposed use of aptamers for the recognition of biomolecules instead of antibodies. Aptamers are simply classified as artificial oligonucleotides that are capable of a wide range of detection of specific biomolecules based upon the aptamer configuration. One attractive appeal of an aptamer approach is that they are less costly and are capable of reversible denaturation meaning that the biosensor can be reused continuously.

The same paper also discusses the detection of prostate specific antigens by anchoring prostate specific antigen antibodies to a nanowire or nanotube surface. These function as specific recognition groups for the prostate specific antigen.

Examples of different elongate nanostructures and their preparation

a) Nanotubes,_e.g. carbon nanotubes, these can be obtained from suppliers, such as Unidym Inc. (purified HiPco). Alternatively, they can be grown using e.g. a chemical vapor deposition (CVD) process in which a substrate such as a silicon wafer is prepared with a layer of catalyst particles, e.g. using group VIII metals as catalyst centers. The catalyst centers then act as growth cites for the carbon nanotubes. During the growth the substrate is heated to a relatively high temperature e.g. 700° C to 900°C and a process gas (such as ammonia, nitrogen or hydrogen) and a carbon containing gas (such as acetylene, ethylene, ethanol or methane) is typically introduced into the CVD chamber. The carbon-containing gas is broken apart at the surface of the catalyst particle, and the carbon is transported to the edges of the particle, where it forms the nanotubes. The catalyst particles can stay at the tips of the growing nanotube during the growth process, or remain at the nanotube base, depending on the adhesion between the catalyst particle and the substrate as is known to the person skilled in the art.

b) One can also grow nanotubes on an un-homogenous quartz substrate. The nanotubes will then grow on the edges of the un-homogenous quartz substrate following which they can be transferred to e.g. the glass substrate 10. Most single-walled nanotubes (SWNT) have a diameter of close to 1 nanometer, with a tube length that can be many millions of times longer. The structure of a SWNT can be conceptualized by wrapping a one-atom-thick layer of graphite called graphene into a seamless cylinder.

c) Nanowhiskers, e.g. Si nanowires, can be produced using the so-called vapor-liquid-solid method (VLS), which is a mechanism for the growth of one-dimensional structures, such as nanowires, from chemical vapor deposition (CVD). The VLS mechanism is typically described in three stages:

- Preparation of a liquid alloy droplet upon the substrate from which a wire is to be grown;
- Introduction of the substance to be grown as a vapor, which adsorbs on to the liquid surface, and diffuses in to the droplet;
- Supersaturation and nucleation at the liquid/ solid interface leading to axial crystal growth.

d) A further mechanism for growing polymer nanowires is known, in which the polymer nanowires are grown in solution by self assembly.

Typical types of elongate nanostructures are: Carbon nanotubes, Silicon nanowires, Germanium nanowires, Indium Arsenide nanowires, Zinc Oxide-based nanowires/nanotubes/nanostructures, Gallium arsenide nanowires, Indium Tin Oxide nanowires / nanotubes, Molybdenum nanowires which can be produced by reducing $MoO_2$ deposited on graphite, graphene-based nanostrucutures (graphene nanowires, graphene nanoribbons, monolayer/bilayer/multilayer graphene), metallic nanowires (Au, Ag, Pd, Pt, Co, Ni, Al etc.).polyaniline-based nanowires, polypropylene-vinylene (PPV) nanowires and other polymer-based nanowires/nanotubes.

As mentioned, the nanostructures can either be produced in situ on the respective electrode before the well is applied to the substrate using one of the above mechanisms to form a sensor. Using one of the electrodes 11, 12 as the growth substrate and applying e.g. a catalyst particle to the electrode 11, 12 to initiate the growth of the nanotube across the gap 14. Alternatively, nanostructures can be grown on a separate substrate and subsequently be transferred to the electrode of the sensor or a nanostructure can simple be transferred from a purchased solution containing the desired nanostructures to an electrode to form the desired sensor.

Single-wall carbon nanotubes as obtained from Unidym Inc. (purified HiPco) are dispersed in a surfactant solution. Subsequently, the nanotubes are trapped in the electrode locations from the dispersion using AC dielectrophoresis. The use of photolithography and the dielectrophoretic trapping ensures that we have a high throughput (> 90%) for obtaining devices. On the average 5 tubes are trapped across the electrodes in every electrode gap. In order to ensure that only the nanotubes are in contact with the liquid, the electrodes are passivated by depositing silicon oxide. The liquid is delivered on to the sensor chip using a microwell 37 that is fixed on the chip as shown in Fig. 4A and 4B. The microwell 37 is comprised of a microchannel connecting two reservoirs carved out in a polydimethylsiloxane (PDMS) layer 35. The channel is positioned exactly above the electrode gap 14. An Ag/AgCl reference electrode 44 placed in the reservoir 37 acts as the gate electrode.

Further details on the technological background and the underlying problems

The detection of specific nucleic acid sequences plays a vital role in environmental, food, and clinical monitoring and in forensic screening. Nucleic acid assays are mainly based on hybridization of target DNA containing a specific sequence using a complementary probe sequence. Deoxyribonucleic acid (DNA) hybridization is the basis for forensic analysis and for the detection of various infections and genetic disorders. On the other hand, the detection of messenger RNA (mRNA) is important for applications such as in the estimation of the gene expression level. The introduction of real-time Polymerase Chain Reaction (RT-PCR) and microarrays have revolutionized the way in which nucleic acid assays are implemented, enabling the highly sensitive detection of various biomarkers. While these methods enable the detection of a few copies of DNA, they require the amplification of the target sequence. Furthermore, the microarray-based detection requires a labeling step, which affects the time, efficiency and the total costs involved in the complete detection protocol.

The ability to detect extremely low concentrations of DNA would improve the diagnostics of various diseases. The present application includes a report on carbon nanotubes based electrical sensor for DNA oligonucleotides which shows very high sensitivity and selectivity and can be integrated into lab-on-a-chip system. Sensors fabrication is done in a way that it can be easily automated and upscaled. Unlike most of the existing DNA hybridization detection techniques no labeling of the DNA strand is required. Probe DNA covers the CNTs surface and changes of electrical impedance values upon complementary strand binding are measured. The ability to detect DNA down to 100 aM concentration was demonstrated. Linear response was observed at 100aM-1pM DNA concentration range. This application describes the preparation of the DNA biosensor, sensing strategy and performance of the detector.

The present invention is directed amongst other things to a label-free detection strategy without the need for PCR to detect a few copies of oligonucleotide target sequences. The active elements in the sensors under discussion here comprise one to ten single-wall carbon nanotubes. The detection utilizes sensitive electrical field-effect based sensing coupled with a refined low-noise impedance measurement. Utilizing a simple fabrication strategy it has been possible to demonstrate the routine fabrication of nanoscale DNA sensors with ultra-high sensitivity down to attomolar concentrations. The ability to detect a few copies of DNA without the need for a bulky optical reading instrument will pave way for the facile entry of nanodiagnostic devices for point-of-care diagnostics.

Sensors based on hybridization of nucleic acids constitute the basis for today's molecular diagnostics. Such sensors find application in the detection of various diseases, depending on the presence or absence of specific nucleotide sequences called biomarkers. In order to detect very low concentrations of such biomarkers or target sequences, the sample is amplified using PCR. For applications involving comparison of gene expression levels, microarrays are used. This requires the labeling of the target sequences to enable their subsequent detection using a fluorescence microscope. While optical methods are currently well-established for the detection of oligonucleotide sequences, they still suffer from the fundamental limit imposed by the diffraction-limited spot size. The need for sample amplification and the requirement of a bulky optical reading instrument limits the use of such sensors for point-of-care applications. Towards this purpose, it is important to reduce the number of preprocessing steps before detection and, in addition, to motivate the need for a portable device that is cost-effective.

Electrical methods are ideally suited for this purpose, since they do not require the target to be labeled. Label-free electrical detection of DNA has been demonstrated in a broad range of research works published until now. The majority of them are based either on field-effect based detection or on electrochemical detection. While the use of a label is avoided in these experiments, the limit of detection is comparable to that of optical methods. As a result of this, these methods also require an amplification step before they can be deployed. In order to improve the limit of detection, nanostructures have been proposed as suitable alternatives as active elements of biosensors. Nanostructures possess a high surface-to-volume ratio and are expected to yield much higher sensitivities than microscale or conventional sensors.

One-dimensional nanostructures (1D-NS) are ideally suited for this purpose since they can be used as active elements of field-effect transistors in a facile manner. Furthermore, a 1D-NS such as a carbon nanotube has all atoms on its surface and every atom limits the current flowing through it. Hence they can be deployed as highly sensitive detectors. Other candidates include semiconducting nanowires such as silicon nanowires. With silicon nanowires the smallest diameter sensors that have been demonstrated until now is of the order of 10 nm. Carbon nanotubes on the other hand have a much smaller diameter of 1 nm. The subsequent discussion of the complex impedance measurement technique and the measurement presented show that with CNT-based electrical detectors one can obtain a close-to-absolute sensitivity, whose limit of detection is one order of magnitude lower than that has been shown with silicon nanowires.

In order to be viable it is important to obtain sensors in a routine manner. While with semiconductor nanostructures it is rather straightforward to obtain nanostructures with similar semiconducting properties with a certain tolerance, in the prior art nanotubes were considered to suffer from a major hurdle. Since nanotube production methods always yield a mixture of metallic and semiconducting tubes. In the past it was thought that to reliably reproduce sensors it was important to have semiconducting tubes exclusively. While there are many methods emerging to obtain tubes of a certain electronic structure, they suffer from very short lengths (of the order of 1 micron or less). While it is possible to realize electrical devices using such short tubes, it will require electron-beam lithography for fabrication. This will in turn reduce the possibility of scalability. In order to be scalable, and in this manner retain the capability of entering the market it is important to deploy photolithography during the fabrication procedure. In such a scenario only long tubes can be used which almost always come as a mixture.

In accordance with the invention it has been found that, for the application as an FET, the metallic nanotubes 32 are actually the preferred semiconducting material used to connect the source and the drain electrode 11, 12 of an FET. This is because metallic nanostructures, such as metallic carbon nanotubes 32, also exhibit semiconducting properties. In contrast to semiconducting nanotubes 32, however, the metallic nanotubes 32 cause an even smaller change in the resistance, i.e. a smaller change in the current flowing between the source and the drain electrode on application of a gain voltage $V_G$ compared to non-metallic nanostructures. The fact that the current passing through the metallic nanostructures only varies minimally on application of the gain voltage is subsequently exploited, as the currents output by the sensors are no longer as sensitive to changes in the gain voltage (e.g. due to temperature fluctuations), but are now more sensitive to changes in the resistance of a solution present in the well 37 of the present invention. This is due to the fact that metallic nanotubes show a slight gate modulation due to the presence of defects. Furthermore, metallic nanotubes show very low resistances in the range of 100 kOhms and are hence ideally suited as highly sensitive sensors.

The detectors are fabricated using these tubes that are dispersed in an aqueous surfactant solution. The tubes are trapped onto photolithographically patterned electrodes using AC dielectrophoresis. In order to obtain absolute sensitivity, it is important to have only the active element in contact with the solution. If this is not guaranteed, the non-active components of the detection system will also contribute to the sensor response. In such a situation the major advantage of 1D-NS, which is high surface-to-volume ratio is completely lost. In order to achieve this, two important steps are necessary. The first step involves the passivation of the electrodes after device fabrication. Here photolithography is used again to cover the electrode areas with silicon dioxide. As a consequence of this, only the nanotubes are in contact with the solution, which is an ideal basis for attaining absolute sensitivity. Moreover, DNA hybridization is detected directly in solution. For this purpose a reference electrode is used that is in contact with the solution as the gate in order to tune the conductivity of the nanotube. Such reference electrodes are shown as 44 and 44' in Figs. 1H, 1J, 2 and 3.

The second important aspect concerns the efficient coupling of the probe sequences to the nanotube. While spotting a solution of probe sequence without any specific chemistry will lead to attachment of the nucleotides on to the CNT surface, it does not exclude the presence of nucleotides on the surface around the CNTs. While this will not have any detrimental effect on the sensor response, the presence of such unwanted sequences will lead to the binding of target sequences there. These binding events will not contribute to the signal and hence the limit of detection will necessarily increase. In order to avoid this an optimized electrochemical functionalization is used. This method allows the direct coupling of receptors on to the surface of the CNTs without any unwanted deposition around the CNTs. This ensures that the probe sequences are immobilized exclusively on the nanotube surface and hence guarantees a very low limit of detection. It is also now well-documented that spotting a DNA directly on to a nanotube would lead most likely to non-specific wrapping of the nanotube. This is expected due to the strong hydrophobic nature of CNTs coupled to the ease of hydrogen bonding by the bases. In order to avoid this, the invention takes measures to render the surface negatively charged and hydrophilic using non-covalent functionalization of the NT surface with carboxylic groups as shown in Fig. 5.

Complex Impedance Measurement

One important element of the present invention is the recognition that it is necessary to carry out a complex impedance measurement in order to sensitively detect analytes because changes in both the magnitude and the phase of the impedance jointly serve as a recognition that an analyte is present and the measurement of both parameters significantly enhances the sensitivity of the detection/measurement. One way to sensitively detect analytes is to measure and analyze the complex impedance of the single wall carbon nanotube or of the elongate nanostructures for a range of different frequencies and for a range of different gate voltages.

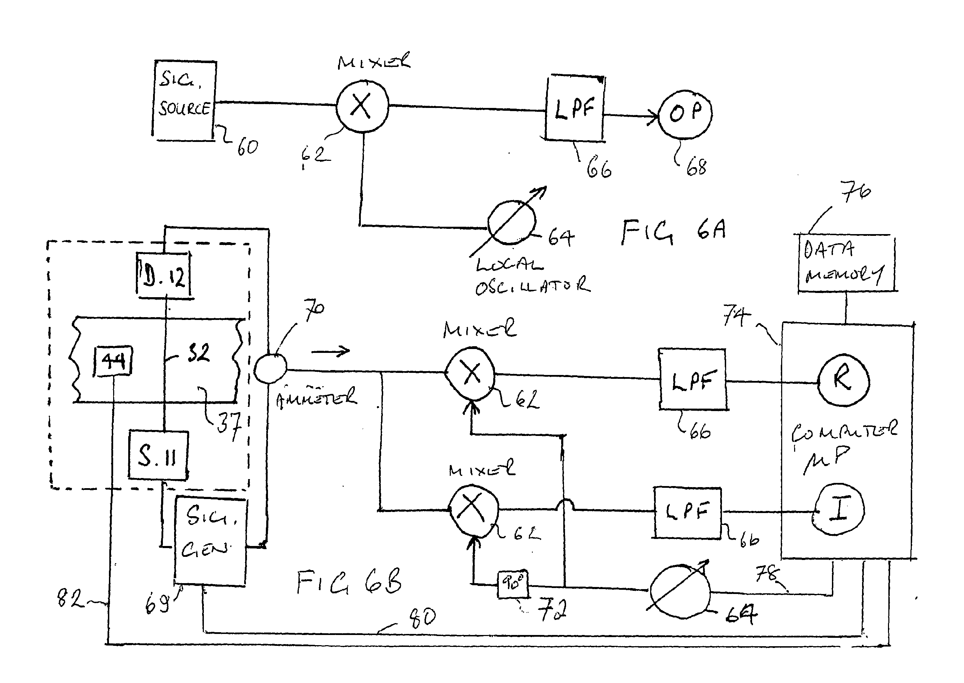

Turning now to Fig. 6A, a measurement circuit will be described which is actually embodied twice in the actual complex impedance analyzer of the invention shown in Fig. 6B.

In Fig. 6A, reference numeral 60 denotes a signal source which provides a frequency signal f comprising a plurality of superimposed sinusoidal signals of different discrete frequencies $f_0$, $f_1$, ...$f_n$, which may, for example but without any intended restriction, comprise 10 different frequencies. This composite signal is applied to one input of a mixer 62.

This input signal can be expressed as:

$$F(t) = A_0 e^{if_0 t} + A_1 e^{if_1 t} + A_2 e^{if_2 t} + ... + A_n e^{if_n t}$$

The second input of mixer 62 is connected to a local oscillator which can be triggered to generate a local oscillator signal of frequency $f_L$, with the frequency $f_L$ being variable and being able to be scanned through a range of frequencies from $f_0$ to $f_n$, for example with the frequency of the local oscillator being set to one of these discrete frequencies $f_0$ to $f_n$ in turn.

The signal of the local oscillator can be expressed as:

$$F_L(t) = e^{ef_L t}$$

In known manner, the output of the mixer 62 is a complex signal comprising the frequencies $f_0 + f_L$, $f_1 + f_L$, $f_2 + f_L$.....fn + fL and $f_0 - f_L$, $f_1 - f_L$, $f_2 - f_L$,....$f_n - f_L$. The output of the mixer 62 is fed to a band pass filter 66.

The sum frequencies $f_0 + f_L$, $f_1 + f_L$, $f_2 + f_L$.....$f_n + f_L$ (which have the amplitudes $A_0$, $A_1$, $A_2$, ..., An) are much higher than the pass band of the band pass filter 66 and thus do not pass through the filter.

For any discrete frequency $f_L$ there will always be one difference frequency from the output $f_0 - f_L$, $f_1 - f_L$, $f_2 - f_L$,....$f_n - f_L$ of the mixer 62 which lies within the pass band of the band pass filter 66, i.e. for example $f_2 - f_L$ = say 10 kHz when $f_L$ is adjusted accordingly. Alternatively a low pass filter can be used which will allow a DC signal, i.e. one with a frequency zero to pass through the low pass filter. This means that when e.g. $f_L = f_1$ the resulting difference frequency is zero and the output of the low pass filter will be $A_1$. Similarly, if e.g. $f_x$ -$f_N$ where $f_L = f_x$ then the output of the low pass filter will be $A_x$.

Another example would be that $f_L$ is adjusted such that $f_n - f_L$ is again, say, 10 kHz. Thus, a band pass filter set to transmit a frequency of 10 kHz has at its output 68 a signal of a frequency, in this example, of 10 kHz, which contains information on the input signal at the respective frequency $f = f_0$ to $f_n$. The output of the band pass filter at any one time is, however, a 10 kHz signal (in this example), but contains the information that was present in a signal at the respective selected higher frequency signal, i.e. at any one of the frequencies $f_0$ to $f_n$. As another specific example, the frequency $f_L$ of the local oscillator is adjusted to be substantially equal to one of the frequencies of the input signal, i.e. $f_L = f_0$, $f_1$, $f_2$, ... $f_n$, so that one of the output difference frequencies $f_0 - f_L$, $f_1 - f_L$, $f_2 - f_L$, ... $f_n - f_L$ of the mixer 62 is always substantially equal to zero, hence providing a DC component at the output of the mixer 62, the amplitude of which contains information about the signal component of the input signal at the respective frequency $f_0$, $f_1$, $f_2$, ... $f_n$. The pass band of the band pass filter can then be adjusted such that only the DC component is transmitted to the output 68.

Turning now to Fig. 6B there is shown a schematic diagram of how the principle described above with reference to Fig. 6A is incorporated in a complex impedance measurement in accordance with the present invention.

To the left of Fig. 6B there is shown schematically (in similar manner to Fig. 4B) an area of a chip showing the source electrode 11, the drain electrode 12, a SWCNT 32 extending between the source electrode 11 and the drain electrode 12, a reference electrode 44 and a section of the well 37 in the insulating layer.

A voltage generator 69 of Fig. 1 now feeds a mixed frequency voltage signal comprising a plurality of superimposed sinusoidal signals of discrete frequencies $f_0$, $f_1$, ...$f_n$ into the source electrode 11 and the return path of the drain electrode 12 passes through an ammeter 70. The ammeter 70 serves as a signal source to two measurement circuits as described above with reference to Fig. 6a. Accordingly, the output signal of the ammeter 70 is fed as an input signal to two mixers 62, both of which receive at their second inputs a signal from the local oscillator 64. However, one of the mixers 62, the lower one in Fig. 6B, receives the signal from the local oscillator 64 after it has been phase shifted by 90 degrees by the phase shifter 72. The outputs of the two mixers 62 are fed to respective band pass filters 66 of the same design. As a result of the phase shift of 90 degrees applied to the signal from the local oscillator 64, the output of one of the band pass filters, the upper one in Fig. 6B, is the real part of the current, whereas the output of the second band pass filter, the lower one in Fig. 6B, is the imaginary part of the current. These two output signals are received by a microprocessor or computer 74 which is connected to a data memory 76 and usually also to a keyboard and a display screen (not shown) which can be used for entering data and visualizing data from the complex impedance measurement. The computer 74 has a connection line 78 to the input of the local oscillator 64 which triggers a frequency scan through the discrete frequencies $f_0$ to $f_n$ at the local oscillator. The computer 74 also has a connection line 80 to the voltage generator 69 which sets the voltage generator into operation.

The computer 74 also has a connection 82 to the reference electrode 44 enabling different voltages to be applied to the reference electrode 44 which acts as a gate for the FET formed by the source electrode 11, the SWCNT 32, the drain 12 and the gate 44 during the complex impedance measurement.

Since the computer 74 is connected to the voltage generator 69 and knows the amplitude in volts of the signal applied by the voltage generator to the source electrode and since the computer 74 also knows the current flowing through the SWCNT 32 from the output signal of the ammeter, and indeed the real and imaginary components of the signal (R and I) it is able to calculate the magnitude and phase of the complex impedance measurement for each discrete frequency of the local oscillator 64.

Moreover, the computer 74 is able to repeat these measurements for different gate voltages applied to the conductive channel formed by the SWCNT 32 by the reference electrode/gate electrode 44.

Although in Fig. 6B the signal generator is shown connected directly to the first and second source and drain electrodes 11 and 12 and the computer is shown connected to the reference electrode 44 acting as a gate, in actual fact the connections are not made directly to the first and second electrodes, but rather to the respective contact pads 24 and 26 and equally the gate potential is not applied directly to the reference electrode 44, but rather to its associated reference pad 48 (24, 26 and 48 are not shown in Fig. 6B, but rather in Figs. 1C, 1J and 2.

It should be noted that the complex impedance measurement is not restricted to the embodiment of Fig. 1J but can also be applied to the embodiment of Fig. 2 (for each SWCNT or other elongate nanostructure in turn) and it can also be used for the embodiment of Fig. 3, but here the measurement is carried out with the signal generator being connected to the first electrode 11 and to one of the reference gates 44, with the gate bias being applied to the same or the other reference electrode 44.

The use of impedance measurement enables the acquisition of both magnitude and phase spectra, and hence a direct estimation of both the resistive and capacitive behavior of the sensor. Furthermore, the use of AC measurement improves signal-to-noise ratio and thereby stability and reproducibility in comparison to a DC measurement. This frequency response is measured at varying gate voltages to characterize the field-effect behavior. The resulting dataset can be visualized in the form of *magnitude Z-maps* and *phase Z-maps* (see e.g. Figs. 8A to 8F). The use of high frequency detection ensures a very low noise and coupled with the stable Ag/AgCl reference electrode provides for excellent stability. This enables the reuse of the same sensor for a series of different DNA concentrations with minimal drifts.

Result of the complex impedance measurement for a specific analyte containing DNA

A typical measurement result can be seen in a 3-dimensional representation, a so-called carpet plot, from Figs. 7A and 7B and a specific measurement result in a more easily compared 2-dimensional form in Figs. 8A to 8F.

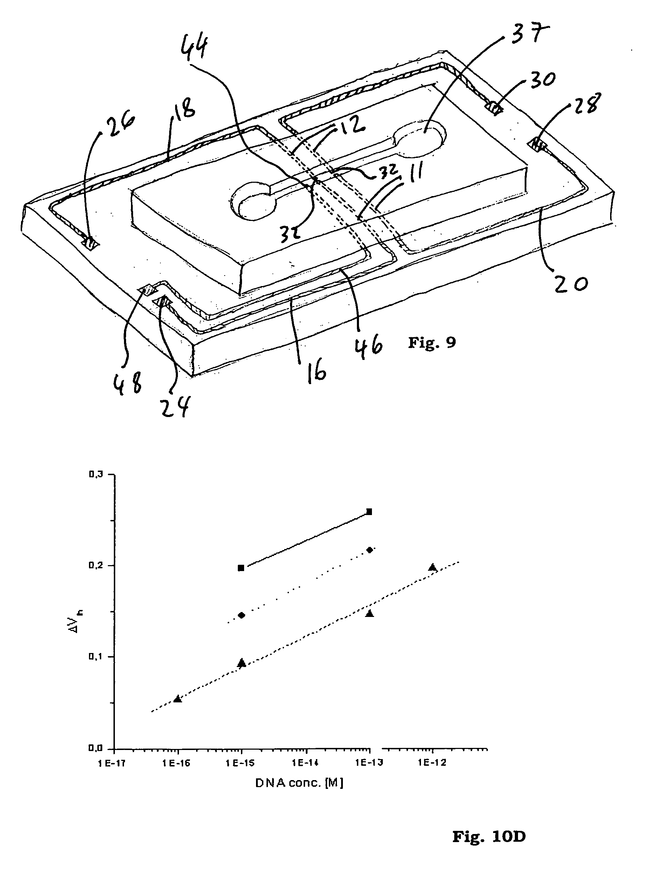

Fig. 9 shows a schematic representation of a detector 10 in accordance with the invention. The detector 10 has two first electrodes 11, two second electrodes 12 and one reference electrode 44. Each electrode 11, 12 is connected via a respective conductive trace 16, 18, 20, 22 to a respective readout pad 24, 26, 28, 30. In this case the reference electrode 44 is connected via a respective trace 46 to a respective contact pad 48 so that bias voltages or measurement voltages can be applied to the reference electrode as required. The dielectrophoresis technique described with reference to Figs. 4A and 4B was used to attach multiple single-wall carbon nanotubes 32 to respective electrodes 11 in Fig. 9 so that the carbon nanotubes extend generally radially away from the first electrodes 11. In the insulating layer 35 there is formed a well 37 which is generally of elongate dumb-bell shape, with the channel between the two generally circular ends of the well 37 standing transverse to the single-wall carbon nanotubes 32 and generally being of a width corresponding to slightly less than the separation between the first and second electrodes 11 and 12.

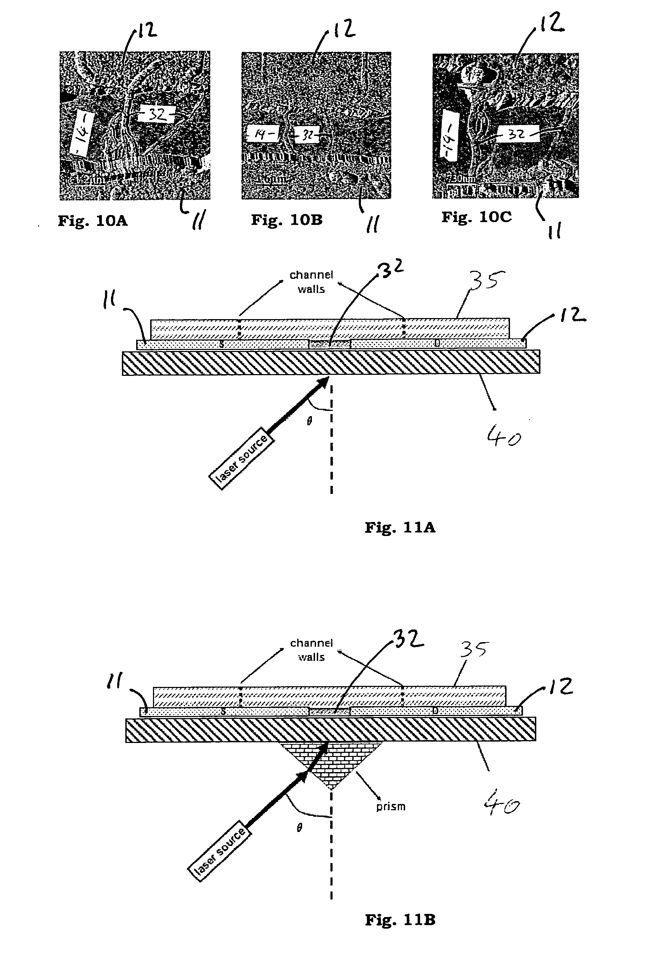

Figs. 10A to 10C show AFM images of various detectors in accordance with the invention. The AFM image of Fig. 10A shows a relatively large bundle of nanotubes 32 and a relatively small long nanotube 32 extending between the first and second electrodes 11, 12. In contrast hereto the AFM image of Fig. 10B shows two relatively thin nanotubes 32 extending between the first and second electrodes 11, 12 of a detector 10. The AFM image of Fig. 10C only shows a relatively large nanotube 32 extending between the first and second electrodes 11, 12. The different size and number of nanotubes 32 extending between the first and second electrodes 11, 12 naturally leads to different sensor responses. The different sensor responses are shown in the graph of Fig. 10D.

Fig. 10D shows the different calibration curves for the different sensors 10 of Figs. 10A to 10C (▲Fig. 10A; ♦Fig. 10B; ■Fig. 10C;). The different calibration curves show the threshold shift as a function of DNA concentration. As can be seen from Fig. 10D the different detectors show a linear response over a broad concentration range. The data was measured with 30 $\mu$L of sample solution in the microwell 37. This corresponds to 1800 molecules of DNA detectable at a concentration of 100 aM. Since the curves of the different sensors 10 show a linear response, only a single test measurement is required to find out a calibration factor for the individual sensor 10 before this can be used to detect what substance is present in the well 37 of the sensor 10.

The entire detection strategy can be augmented with a source of optical excitation in order to improve the detection sensitivity and / or to extend the range of analytes that can be detected. For example, using the method described before, uncharged analytes which do not produce any charge or permittivity differences cannot be detected easily. Using the augmented method with laser excitation it is possible to sensitively detect uncharged species for e.g. steroid hormones such as progesterone in physiological pH. Fig.11A shows a schematic of this augmented setup where a laser source has been added to the embodiment of Fig. 9. The laser optically excites the medium in which the receptor is attached to the carbon nanotubes. The parameters of the laser that can be varied include the wavelength ($\lambda$), intensity ($I_L$), and the angle of illumination ($\theta$). In addition the intensity and / or the wavelength of the exciting laser can be modulated. The sensor signal comprises of magnitude and phase Z-maps similar to the cases described above. The Z-maps measured here (named photo-Z-maps) are a function of frequency and gate voltage and in addition a function of $\lambda$, $I_L$ and $\theta$. The binding or non-binding of the analyte to the receptor changes the dielectric properties of the environment around the nanotube. This modifies in turn the measured photoresponse resulting in a dif ferent photo-Z-map. Possible mechanisms for this change include but not restricted to photoconductivity, photothermal effects, photovoltage generation, photodesorption, plasmons, etc. This change is used to identify the presence or absence of the analyte and to quantify it. The laser source could be provided by a laser diode or a gas laser or a tunable laser or a focussed beam from a confocal microscope or mercury/halogen lamps or similar sources. The light could be coupled to the substrate directly as shown in fig.11A or through a prism as shown in fig. 11B. The prism is useful in the dedicated excitation of surface plasmons (through total internal reflection), which in turn will induce changes in photo-Z-maps when the analyte interacts with the receptors on the nanotube surface. The well shown in figures 11A and 11B can be open or closed. In the latter case, a flow channel could be constructed in the form of a closed system, where the analytes can be controllably brought on to the surface of the sensor. The laser excitation need not necessarily come from below; the sample can also be illuminated from the top. In the case of a closed system, the sample can also be turned upside down. The laser excitation itself can also be integrated on-chip.

One possible reason for the change in impedance and/or the dielectric properties is due to the fact that the laser introduced into the well creates plasmons which are collective oscillations of the free electron gas density, e.g. at optical frequencies. Plasmons play a large role in the optical properties of metals. Light of frequency below the plasma frequency is reflected, because the electrons in the metal screen the electric field of the light. Light of frequency above the plasma frequency is transmitted, because the electrons cannot respond fast enough to screen it. In most metals, the plasma frequency is in the ultraviolet, making them shiny (reflective) in the visible range.

In semiconductors, the valence electron plasma frequency is usually in the deep ultraviolet, which is why they too are reflective. Position and intensity of plasmon absorption and emission peaks are affected by molecular adsorption, which can be used in molecular sensors. For example, a fully operational prototype device detecting casein in milk has been fabricated. The device is based on detecting change in absorption of a gold layer. Localized surface plasmons of metal nanoparticles can be used for sensing different types of molecules, proteins, etc.

The method of preparing the sensors, the functionalization of the SWNCTs with DNA and the trials carried out will be described in the following using the inventors terminology

*Fabrication of sensors.* Single-wall carbon nanotubes (SWCNTs) were dispersed with the help of sonication in 0.1% Triton X-100. Undispersed material was removed using centrifugation for 1h at 4600g, followed by filtration. Using dielectrophoretic force, the tubes were positioned across preformed platinum electrodes on $SiO_2$ surface. The parameters used for dielectrophoresis were following: 10 MHz, 10 V, 15 s. Electrodes were passivated with 200 nm $SiO_2$ using confocal lithography [38]. To improve nanotube-metal contact, samples were annealed in argon at 600° C for 45 s. Following this, the samples were placed on a chip carrier and wire bonded to the leads. A microwell was prepared by manually carving out reservoirs and a microchannel in a polydimethylsiloxane (PDMS, Sylgard 184, 10:1) layer. The microwell was stuck to the chip surface after wire bonding. The samples were characterized using an atomic force microscope (AFM) and electronic transport measurements. An Ag/AgCl reference electrode placed in contact with the liquid in the microfluidic circuit allows us to set the potential precisely on the surface of the nanotube. This electrode serves two purposes namely as a reference electrode for electrochemical functionalization and as a gate electrode for measuring the sensor characteristics.

*Functionalization of SWCNTs with probe DNA.* The contacted carbon nanotubes were functionalized with 4-aminobenzoic acid via oxidative electropolymerization. For this purpose, a solution of 4-aminobenzoic acid and $LiClO_4$ in ethanol was added to the microwell and the voltage at the nanotubes was swept from -0.1 to +0.7V against Ag/AgC1 reference electrode. After 5 sweeps, the chip was carefully washed with ethanol and water. Single stranded $NH_2$-DNA (MWG) was attached to carboxyl groups of polymerized aminobenzoic acid by carbodiimide coupling. For this purpose, the well was filled for 15 min with a mixture of ethylenediaminechloride (EDC) and N hydroxysuccinimide (NHS) 0.5 M (1:1) in phosphate buffer for activation of carboxyl groups. After that most of the solution was taken out from the well and it was filled with $NH_2$-DNA in the same buffer for 30 min. Ethanolamine was used to block remaining activated carboxyl groups. The well was subsequently washed with the same buffer. Before the sensor trials, several measurements are taken to ensure stable electrical properties of device in buffer solution and also to limit the amount of possible non-specifically absorbed probe DNA. In order to detect a specific target DNA sequence through hybridization, a complementary probe sequence needs to be immobilized on the nanotube surface. Towards this purpose a versatile electrochemical functionalization route was utilized in which a range of sensors based on nanotubes and grapheme were realized.

*DNA Sensing Trials.* The sensing trials were performed on more than 10 devices using varying concentrations of DNA in 10 mM potassium phosphate buffer with O.1M NaCl (pH 7.4). The impedance (Z, a complex quantity with magnitude and phase) constitutes the sensor response, which was measured using an impedance analyzer (Agilent 33250A LCR Meter) in a frequency range of 20 Hz to 2 MHz. This frequency response is measured at varying gate voltages to characterize the field-effect behavior. The gate voltages are maintained in the range of -0.4 to 0.2 V (at the Ag/AgCl reference) to ensure that no parasitic electrochemical processes can take place. The resulting data set can be visualized in the form of a magnitude map as shown in figure 2. The X-axis corresponds to the liquid gate voltage, while the Y-axis indicates frequency. Every point in the image corresponds to the magnitude of impedance at a certain frequency and gate voltage. The DNA sequences used in this study : probe DNA: 5-ggcctcacgtcacactctccgcgc-3, target DNA: 5-gcgcggagagtgtgacgtgaggcc-3, 3-basepair-mismatched DNA: 5-gcgagcagagggtgacgtgaggcc-3.

**Claims**

1. An apparatus for detecting one or more analytes, for example analytes selected from the group comprising nucleic acids, metabolites, peptides, proteins, hormones, pesticides, neurotransmitters, ions in blood, electrolytes, toxic gases, pH and biological warfare agents, the apparatus comprising an insulating substrate, at least one first electrode on the substrate at least one elongate nanostructure extending from and electrically connected to the or each said electrode and extending over the surface of the wafer away from the respective electrode, a passivating layer covering the or each electrode, but not all of said at least one elongate nanostructure, a well crossing the at least one elongate nanostructure extending from the or each electrode and forming a static reservoir for a liquid being investigated for the presence of at least one analyte, a reference electrode provided on said substrate within said well or insertable into said well and respective readout pads electrically connected to the or each electrode and to the reference electrode if the latter is provided on the substrate, the at least one elongate nanostructure being capable of being functionalized for detecting one or more analytes.

2. An apparatus in accordance with claim 1, wherein at least one layer of insulating material overlies the or each electrode and at least part of the substrate and said well is provided in said insulating layer.

3. An apparatus in accordance with claim 1 or claim 2 and further comprising at least one second electrode provided on said substrate and spaced from a said first electrode by a gap, said at least one elongate nanostructure extending between and electrically connected to a first electrode and a second electrode across said gap, said passivating layer covering each of said first and second electrodes, but not all of said at least one elongate nanostructure, the at least layer of insulating material overlying the first and second electrodes and the well crossing the at least one elongate nanostructure in the gap and forming a static reservoir for a liquid being investigated for the presence of at least one analyte, a reference electrode provided on said substrate within said well or insertable into said well and readout pads electrically connected to the first and second electrodes and optionally to the reference electrode if provided on the substrate, the at least one elongate nanostructure crossing said gap being capable of being functionalized for detecting one or more analytes.

4. An apparatus in accordance with claim 3, there being a plurality of pairs of first and second electrodes on the same substrate, each pair of first and second electrodes being spaced apart by a respective gap and having respective readout pads, respective elongate nanostructures crossing said gaps, the one-dimensional nanostructure or nanostructures crossing said gap being respectively functionalized for the one or more analytes, there being a common well extending over all said gaps or a respective well for each said gap or for a group of gaps.

5. An apparatus in accordance with any one of the preceding claims in combination with readout circuitry connectable or connected to the respective readout pads and to said reference electrode and adapted to carry out an A/C measurement of complex impedance, i.e. of magnitude and phase, for the or each pair of first and second electrodes respectively.

6. An apparatus in accordance with any one of the preceding claims, wherein said one-dimensional nanostructure or nanostructures is/are selected from the group comprising single-wall carbon nanotubes with metallic and/or semiconducting character, carbon nanowires or a graphene nanoribbon; metallic nanowires, for example of gold or palladium or platinum, polymer nanowires, for example polyaniline (PANI) or polypropylene vinylene (PPV) which have metallic or semiconducting character and inorganic nanowires, such as ZnO, or a combination of any of the above, said nanostructures preferably having a major cross-sectional dimension of not more than 50 nm and especially preferably of not more than 10 nm and most especially preferred of 5 nm or less.

7. An apparatus in accordance with any one of the preceding claims, wherein said layer of insulating material comprises a layer of a material selected from the group comprising: PDMS, polyimide, polyethylene, SU8, glass, $SiO_2$, $SiO_x$, or $Si_3N_4$, and/or wherein said substrate is a material selected from the group comprising glass, polyimide, silicon/silicon oxide, quartz and silicon nitride.

8. An apparatus in accordance with any one of the preceding claims, in combination with an optical excitation source e.g. a laser for optical excitation of a substance present in said well preferably generating surface plasmons in said substance, and / or wherein an optical coupling device is provided at the apparatus, for example, at the rear of the substrate for coupling optical signals into the well in the vicinity of the substance.

9. A method of fabricating an apparatus for detecting one or more analytes, especially an apparatus as claimed in any one of the preceding claims, the method comprising the steps of:

a) fabrication of electrodes on a substrate,
b) deposition, growth or transfer of at least one elongate nanostructures on the substrate with one end contacting a respective electrode, with step b) being carried out before or after step a)
c) deposition of a passivating layer over the or each electrode contacted by the one-dimensional nanostructures,
d) forming a well therein extending across said one-dimensional nanostructure or nanostructures either in the substrate or in an insulting layer over the electrodes and the substrate
e) providing a respective readout pad connecting to each said at least one electrode and/or
f) electrochemically treating said nanostructures and/or
g) functionalizing said nanostructures with a receptor or receptors specific to an analyte.

10. A method in accordance with claim 9 and comprising fabrication of at least one first electrode and at least one second electrode on said substrate, said at least one second electrode being spaced from said first electrode by a gap, and deposition or growth of at least one elongate nanostructure such that the or each elongate nanostructure has a first end contacting said first electrode and a second end contacting said second electrode, and forming said well across said gap.

11. A method in accordance with claim 9 or claim 10 and including the step of forming a reference electrode on said substrate at a position such that it lies within said well.

12. A method in accordance with any one of the claims 9 to 11 including the step of forming a plurality of pairs of first and second electrodes on the same substrate, each pair of first and second electrodes being spaced apart by a respective gap and having respective readout pads, and forming a plurality of elongate nanostructures crossing each said gap, and functionalizing the plurality of elongate nanostructure or nanostructures crossing said gap for the one or more analytes, and providing in said insulating layer either a common well extending over all said gaps or a respective well for each said gap or for a group of gaps.

13. A method in accordance with any one of the preceding claims 9 to 12 and including the further step of connecting readout circuitry to the respective readout pads and to the or a reference electrode said readout circuit being adapted to carry out an A/C measurement of complex impedance, i.e. of magnitude and phase, for the or each pair of first and second electrodes respectively.

14. A method in accordance with any one of the preceding claims 9 to 13, wherein said elongate nanostructure or nanostructures is/are selected from the group comprising single-wall carbon nanotubes with metallic and/or semiconducting character, carbon nanowires, graphene nanoribbons, metallic nanowires, for example of gold or palladium or platinum, polymer nanowires, for example polyaniline (PANI) or polypropylene vinylene (PPV) which have metallic or semiconducting character and inorganic nanowires, such as ZnO, or a combination of any of the above, said nanostructures preferably having a major cross-sectional dimension of not more than 50 nm and especially preferably of not more than 10 nm and most especially preferred of 5 nm or less.

15. A method in accordance with any one of the preceding claims 9 to 14, wherein said layer of insulating material is selected from the group comprising: PDMS, polyimide, polyethylene, SU8, glass, $SiO_2$ $SiO_x$, or $Si_3N_4$, wherein said substrate is selected from the group comprising glass, polyimide, silicon/silicon oxide, quartz and silicon nitride.

Fig. 1A

Fig. 1B

Fig. 1C

Fig. 1D

SiO₂ passivation layer

Borders of channel in PDMS layer

Fig. 1E

19

Fig. 1F

Fig. 1G

Fig. 1H

Fig. 1I

Fig. 2

Fig. 3

**Fig. 4A**

**Fig. 4B**

**Fig. 5A**

EDC

Sulfo-NHS

NH2-DNA

**Fig. 5B**

SIG. SOURCE 60

MIXER

X 62

LPF 66

OP 68

LOCAL OSCILLATOR 64

FIG 6A

DATA MEMORY 76

D.12

44 32

37

70

AMMETER

S.11

SIG. GEN. 69

MIXER

X 62

LPF 66

MIXER

X 62

LPF 66

90° 72

64

74

COMPUTER MP

R

I

78

80

82

FIG 6B

**Fig. 7A**

**Fig. 7B**

# Magnitude Z-map
## contour plot

In Buffer

After
hybridization

After
melting

frequency [Hz]

liquid gate
voltage [V]

Contour labels in kOhms
········guide to the eye

**Fig. 8A**

**Fig. 8B**

**Fig. 8C**

## Phase Z-map
## contour plot

**In Buffer**

**After hybridization**

**After melting**

frequency [Hz]

liquid gate voltage [V]

Contour labels in degrees

········guide to the eye

**Fig. 8D**

**Fig. 8E**

**Fig. 8F**

Fig. 9

DNA conc. [M]

Fig. 10D

**Fig. 10A**

**Fig. 10B**

**Fig. 10C**

**Fig. 11A**

**Fig. 11B**

Europäisches
Patentamt

European
Patent Office

Office européen
des brevets

**EUROPEAN SEARCH REPORT**

Application Number

EP 10 01 2585

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAKASHIMA Y ET AL: "Fabrication Process of Carbon Nanotube FETs Using ALD Passivation for Biosensors", MICROPROCESSES AND NANOTECHNOLOGY, 2007 DIGEST OF PAPERS, IEEE, PISCATAWAY, NJ, USA, 5 November 2007 (2007-11-05), pages 488-489, XP031233340, ISBN: 978-4-9902472-4-9 | 1-7,9-15 | INV. G01N27/414 G01N27/12 B82Y15/00 |
| Y | * the whole document * | 1,5,8,9, 13 | |
| Y | US 2010/087013 A1 (LIEBER CHARLES M [US] ET AL) 8 April 2010 (2010-04-08) * paragraph [0082]; figure 2 * | 1,9 | |
| A,D | US 7 091 096 B2 (BALASUBRAMANIAN KANNAN [IN] ET AL) 15 August 2006 (2006-08-15) * the whole document * | 1-15 | |
| Y | EP 1 736 760 A2 (HARVARD COLLEGE [US]) 27 December 2006 (2006-12-27) * paragraphs [0078], [0083]; figure 1b * | 5,8,13 | TECHNICAL FIELDS SEARCHED (IPC) |
| A | US 2009/152596 A1 (YANG JONG HEON [KR] ET AL) 18 June 2009 (2009-06-18) * the whole document * | 1-15 | G01N B82Y |

The present search report has been drawn up for all claims

| Place of search | Date of completion of the search | Examiner |
|---|---|---|
| Munich | 25 January 2012 | Klein, Marc-Oliver |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 01 2585

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2012

| Patent document cited in search report | | Publication date | Patent family member(s) | | Publication date |
|---|---|---|---|---|---|
| US 2010087013 | A1 | 08-04-2010 | AT | 497023 T | 15-02-2011 |
| | | | AU | 2007309660 A1 | 02-05-2008 |
| | | | CA | 2655340 A1 | 02-05-2008 |
| | | | EP | 2035584 A2 | 18-03-2009 |
| | | | JP | 2009540333 A | 19-11-2009 |
| | | | US | 2010087013 A1 | 08-04-2010 |
| | | | WO | 2008051316 A2 | 02-05-2008 |
| US 7091096 | B2 | 15-08-2006 | NONE | | |
| EP 1736760 | A2 | 27-12-2006 | NONE | | |
| US 2009152596 | A1 | 18-06-2009 | KR | 20090062373 A | 17-06-2009 |
| | | | US | 2009152596 A1 | 18-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7091096 B

### Non-patent literature cited in the description

- **MARIA TERESA MARTINEZ ; YU-CHIH TSENG ; NERES ORMATEGUI ; IRAIDA LOINAZ ; RAMON ERITJA ; JEFFREY BOKOR.** Label-Free DNA Biosensors Based on Functionalized Carbon Nanotube Field Effect Transistors. *Nano Letters,* 2009, vol. 9 (2), 530-536

- **GUODONG LIU ; YUEHE LIN.** *Analytical Chemistry,* 2005, vol. 77 (18), 5894-5901
- **BRETT LEE ALLEN ; PADMARKA D. KICHAMBARE ; ALEXANDER STAR.** Carbon nanotube field-effect transistor-based biosensors. *Advanced Materials,* 2007, vol. 19, 1439-1451